# EUROPEAN PATENT APPLICATION

(11) **EP 4 279 078 A1**
(43) Date of publication of application: **22.11.2023**
(21) Application number: 22750028.7
(22) Date of filing: 04.02.2022
(51) Int. Cl.: A61K 35/22, A61K 38/17, A61P 13/12, C12N 5/071, C07K 14/47, C12Q 1/686, A01K 67/027, G01N 33/569, G01N 33/68

(54) **COMPOSITION FOR PREVENTION OR TREATMENT OF KIDNEY DISEASE**

(30) Priority: 05.02.2021 KR 20210016870
(71) Applicant: NAM, Ki Taek, Gyeonggi-do, 10416 (KR)
(72) Inventor: NAM, Ki Taek, Goyang-si Gyeonggi-do 10416 (KR); LEE, Yura, Seoul 02781 (KR)
(74) Representative: Cabinet Nony
(86) International application number: PCT/KR2022/001727
(87) International publication number: WO 2022/169279

(57) **Abstract**

Kidney tissue-derived stem cells or organoids according to the present invention are easy to apply for treatment, may be supplied in large amounts due to their high self-renewal capacity, have an excellent ability to differentiate into kidney cells, are less likely to form tumors, and have an excellent ability to regenerate damaged tissue when injected directly into lesions. Therefore, they may be used very suitably for regenerative therapy based on adult stem cells among these stem cells. In addition, a composition according to the present invention is capable of specifically selecting only kidney tissue-derived stem cells among kidney cells. Furthermore, kidney tissue-derived stem cells expressing Lrigl protein or a gene encoding the same have excellent self-renewal and pluripotent abilities and are able to differentiate into nephrons, and thus they may be used very effectively for the prevention or treatment of kidney disease.

## Description

### Technical Field

The present invention relates to a composition for preventing or treating kidney disease.

### Background Art

The kidney is an important organ that maintains the homeostasis of the body, regulates the amount of body fluid and the ion concentration and pH in the blood, excretes waste products such as metabolic waste products, toxins, and drugs, regulates blood pressure, and performs other metabolic endocrine functions. In addition, the kidney activates vitamin D, helping calcium to be absorbed in the small intestine, and is also involved in the synthesis of various hormones. Kidney disease refers to a condition in which the kidney fails to normally perform excretion, regulation, metabolism, and endocrine functions, and the overall function thereof is reduced or abnormal. Decreased function of the kidneys due to damage thereto results in enlargement of the kidneys and related structures, atrophy of the kidneys, alterations in body fluid volume, electrolyte imbalance, metabolic acidosis, impairment of gas exchange, impaired anti-infective function, accumulation of uremic toxins, etc. In particular, acute renal failure is an intractable disease with a high mortality rate, which is caused by decreased renal function due to various causes. Even if renal function is restored, acute renal failure may recur depending on the cause and severity of the damage, or progress to chronic and end-stage renal failure if not treated.

Despite the development of modern medicine, many patients admitted to the hospital suffer from a decrease in renal function, and in particular, patients with high severity of the disease require renal replacement therapy due to a decrease in renal function in many cases. The prevalence of acute renal injury has been reported to be from about 5% of hospitalized patients to about 30 to 50% of patients admitted to intensive care units, and this prevalence is steadily increasing despite the development of new therapeutic methods.

In order to treat such kidney diseases, preclinical reports and clinical trials on stem cells are currently being actively conducted. Specifically, various types of stem cells, including mesenchymal stem cells, adipose-derived stem cells, amniotic fluid stem cells and kidney progenitor cells, have been studied in various ways to repair the kidney. Among these stem cells, in particular, cell therapy products using adult stem cells derived from adult kidneys have the potential benefit of improved renal engraftment and differentiation and can be very effectively utilized in autologous therapies.

In this context, in the case of the kidney, it has been reported that adult stem cells exist in the proximal tubular end, glomeruli, and renal papilla, and various studies have been conducted on cell therapy products capable of inducing the regeneration of damaged kidney cells by such stem cells.

However, studies on identification of such kidney stem cells, identification of stem cells for application as therapeutic agents, and the applicability thereof for clinical use are still insufficient.

### DISCLOSURE

### Technical Problem

An object of the present invention is to provide a pharmaceutical composition for preventing or treating kidney disease containing kidney tissue-derived stem cells as an active ingredient.

Another object of the present invention is to provide a method for producing kidney organoids, kidney organoid produced thereby, and a pharmaceutical composition for preventing or treating kidney disease containing kidney organoids as an active ingredient.

Still another object of the present invention is to provide a composition for detecting kidney tissue-derived stem cells, and a kit for detecting kidney tissue-derived stem cells comprising the same.

Yet another object of the present invention is to provide a method for detecting kidney tissue-derived stem cells, and a method for isolating kidney stem cells.

Still yet another object of the present invention is to provide a method for culturing kidney tissue-derived stem cells.

However, objects to be achieved by the present invention are not limited to the above-mentioned objects, and other objects not mentioned herein will be clearly understood by those skilled in the art from the following description.

### Technical Solution

### 1. Pharmaceutical composition containing kidney tissue-derived stem cells as an active ingredient

One embodiment of the present invention provides a pharmaceutical composition for preventing or treating kidney disease containing kidney tissue-derived stem cells as an active ingredient.

The kidney tissue-derived stem cells of the present invention express Lrigl (leucine-rich repeats and immunoglobulin-like domains 1) protein or a gene encoding the same.

Kidney epithelial cells expressing Lrigl or the gene encoding the same according to the present invention are kidney tissue-derived stem cells having stemness, unlike other cells, and in particular, these cells are involved in tubulogenesis, which is the late developmental stage of the kidney, and are able to differentiate into nephrons. Therefore, for the purpose of the present invention, when the kidney epithelial cells expressing Lrigl or the gene encoding the same, which have stemness, are used, it is possible to prevent or treat kidney disease very effectively.

"Lrigl" in the present invention is a transmembrane protein that interacts with receptor tyrosine kinases such as EGFR-family, MET and RET proteins. Lrigl may be derived from mammals including primates such as humans and monkeys, rodents such as mice and rats, and the like, and may be, for example, human Lrigl (a polypeptide encoded by accession number: NM_015541 or NP_056356, or a polypeptide represented by SEQ ID NO: 1), without being limited thereto.

In the present invention, the kidney tissue-derived stem cells may further express Klf6 (Krueppel-like factor 6) protein or a gene encoding the same.

The "Klf6" in the present invention is a protein encoded by the KLF6 gene corresponding to a tumor suppressor gene. The Klf6 may be derived from mammals including primates such as humans and monkeys, rodents such as mice and rats, and the like, and may be, for example, human Lrigl (a polypeptide encoded by accession number: NP_001153596.1 or NM_001160124.1; a polypeptide encoded by NP_001153597.1 or NM_001160125.1 [Q99612-3]; a polypeptide encoded by NP_001291.3 or NM_001300.5 [Q99612-1], or a polypeptide represented by SEQ ID NO: 2), without being limited thereto.

The "kidney tissue-derived stem cells" in the present invention are stem cells present in kidney tissue, and refer to self-renewing and pluripotent stem cells capable of differentiating into all cell types of the kidney. These stem cells may be involved in the regeneration of damaged kidney and the homeostasis of the kidney.

The kidney tissue-derived stem cells of the present invention may be used as a cell therapy product.

The "cell therapy product" in the present invention is living cells that are used in a treatment method in which they are injected directly into a patient, and refers to a pharmaceutical product manufactured by manipulating living autologous cells, allogeneic cells or xenogeneic cells by a physical, chemical or biological method including *in vitro* culture, proliferation or selection of these cells. For the purpose of the present invention, the kidney tissue-derived stem cells express the Lrigl protein present in the patient's kidney or the gene encoding the same, have excellent stemness, and thus have the advantage of having few side effects such as transplant rejection.

The "kidney disease" in the present invention is a disease that causes weakening of kidney function, and examples thereof include acute kidney injury (AKI) or chronic kidney disease (CKD), depending on the rate at which kidney function deteriorates. For example, the kidney disease may be acute kidney injury, without being limited thereto.

The kidney disease in the present invention may be, for example, at least one selected from the group consisting of glomerulonephritis, chronic renal failure, acute renal failure, nephrotic syndrome, pyelitis, kidney stones, and kidney cancer, without being limited thereto.

The "prevention" in the present invention means reducing either the degree of occurrence of pathological cells in animals or damage to or loss of cells. The prevention may be complete or partial. In this case, the prevention may mean a phenomenon in which the occurrence of pathological cells or abnormal immune function in the subject is reduced compared to the case where the composition for preventing and treating kidney disease is not used.

The "treatment" in the present invention refers to any action associated with clinical intervention for the purpose of altering the natural course of the subject or cell being treated, and may be performed either for prophylaxis or during the course of clinical pathology. Desirable effects of the treatment may include preventing occurrence or recurrence of a disease, or alleviating symptoms, or diminishing any direct or indirect pathological consequences of the disease, or preventing metastasis, lowering the rate of disease progression, ameliorating or palliating the disease state, or improving prognosis. That is, the term "treatment" may be interpreted as encompassing any action that alleviates or cures symptoms of kidney disease by the composition.

The kidney tissue-derived stem cells of the present invention may be administered at any one dose selected from among 1 × 10⁷ to 1 × 10⁸, 1 × 10⁸ to 2 × 10⁸, 2 × 10⁸ to 4 × 10⁸, 4 to 10⁸ to 6 × 10⁸, 6 × 10⁸ to 8 × 10⁸, 8 × 10⁸ to 1 × 10⁹, 1 × 10⁹ to 2 × 10⁹, 2 × 10⁹ to 4 × 10⁹, 4 × 10⁹ to 1 × 10¹⁰, 2 × 10⁸ to 6 × 10⁸, 6 × 10⁸ to 1 × 10⁹, 1 × 10⁸ to 2 × 10⁸, 2 × 10⁸ to 2 × 10⁹, 1 × 10⁷ to 1 × 10⁸, 1 × 10⁸ to 1×10⁹, 1 × 10⁹ to 1 × 10¹⁰, and 1 × 10⁷ to 1 × 10⁹ cells/kg, without being limited thereto.

In the present invention, the pharmaceutical composition may be in the form of capsules, tablets, granules, injections, ointments, powders, or beverages, and the pharmaceutical composition may be for administration to humans.

For use, the pharmaceutical composition of the present invention may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may further contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. In addition, the pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition may further contain a filler, an anticoagulant, a lubricant, a wetting agent, a flavoring agent, an emulsifier, a preservative, or the like.

The routes of administration of the pharmaceutical composition according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. For example, the route of administration may be oral or parenteral administration.

In the present invention, "parenteral" includes subcutaneous, intradermal, intravenous, intramuscular, intra-articular, intra-synovial, intrasternal, intradural, intralesional and intra-cranial injection or infusion techniques. For the purpose of the present invention, the pharmaceutical composition may be administered by direct injection into the kidney, without being limited thereto.

The administration dose of the pharmaceutical composition of the present invention may vary depending on a variety of factors, including the activity of a specific compound used, the patient's age, body weight, general health status, sex, diet, the time of administration, the route of administration, the rate of excretion, drug combination, and the severity of a particular disease to be prevented or treated. Although the dose of the pharmaceutical composition varies depending on the patient's condition and body weight, the severity of the disease, the form of drug, the route of administration, and the duration of administration, it may be appropriately selected by a person skilled in the art. The pharmaceutical composition may be administered at a dose of 0.0001 to 50 mg/kg/day or 0.001 to 50 mg/kg/day. The pharmaceutical composition may be administered once or several times a day. The dose does not limit the scope of the present invention in any way. The pharmaceutical composition according to the present invention may be formulated as pills, sugar-coated tablets, capsules, liquids, gels, syrups, slurries, or suspensions.

### 2. Organoids

Kidney organoids, which will be described later in the present invention, are easy to apply for treatment, may be supplied in large amounts due to their high self-renewal capacity, have an excellent ability to differentiate into kidney cells, are less likely to form tumors, and have an excellent ability to regenerate damaged tissue when injected directly into lesions. Therefore, the organoids may be used very suitably for adult stem cell-based regenerative therapy. In particular, these kidney organoids advantageously have excellent direct regeneration effects compared to existing cell therapy products based on mesenchymal stem cells, embryonic stem cells, or dedifferentiated pluripotent stem cells.

Hereinafter, kidney organoids, a method for producing kidney organoids, and the use thereof will be described in detail.

### (1) Kidney organoids

Another embodiment of the present invention provides kidney organoids.

The kidney organoids of the present invention comprise kidney tissue-derived stem cells expressing Lrigl protein or a gene encoding the same.

Kidney epithelial cells expressing Lrigl or the gene encoding the same according to the present invention are kidney tissue-derived stem cells having stemness, unlike other cells, and in particular, these cells are involved in tubulogenesis, which is the late developmental stage of the kidney, and are able to differentiate into nephrons. Therefore, for the purpose of the present invention, the kidney epithelial cells expressing Lrigl or the gene encoding the same, which have stemness, are able to form organoids very effectively.

In the present invention, the kidney tissue-derived stem cells may further express Klf6 protein or a gene encoding the same.

In the kidney organoids of the present invention, contents regarding the Lrigl or Klf6 protein or the gene encoding the same, the kidney tissue-derived stem cells, etc. are the same as described above in "1. Pharmaceutical composition containing kidney tissue-derived stem cells as an active ingredient", and thus the description thereof will be omitted.

The "organoid" in the present invention refers to cells having a 3D structure, and refers to a tissue-like model produced through an artificial culture process without being collected or obtained from an animal or the like. Unlike 2D culture, 3D cell culture allows cells to grow in all directions *in vitro.*

### (2) Method for producing kidney organoids

Another embodiment of the present invention provides a method for producing kidney organoids.

The method for producing kidney organoids according to the present invention comprises steps of: (a) isolating cells expressing Lrigl (leucine-rich repeats and immunoglobulin-like domains 1) protein or a gene encoding the same from kidney epithelial cells isolated from a subject of interest; (b) culturing the cells expressing the Lrigl protein or the gene encoding the same; and (c) forming organoids from the cultured cells in Matrigel.

The kidney epithelial cells expressing Lrigl or the gene encoding the same according to the present invention are kidney tissue-derived stem cells having stemness, unlike other cells, and in particular, these cells are involved in tubulogenesis, which is the late developmental stage of the kidney, and are able to differentiate into nephrons. Therefore, for the purpose of the present invention, when the kidney tissue-derived stem cells expressing Lrigl or the gene encoding the same, which have stemness, are used, it is possible to produce kidney organoids in a very high yield.

In addition, the cells isolated in step (a) of the present invention may express Klf6 (Krueppel-like factor 6) protein or a gene encoding the same.

In the method for producing kidney organoids according to the present invention, contents regarding the Lrigl or Klf6 protein or the gene encoding the same, the kidney tissue-derived stem cells, the organoids, etc. are the same as described above in "1. Pharmaceutical composition containing kidney tissue-derived stem cells as an active ingredient" and "(1) Kidney organoid", and thus the description thereof will be omitted.

In the present invention, step (a) of isolating the cells may be performed through magnetic activated cell sorting (MACS) or flow cytometry analysis according to a conventional method after dissociating the sample isolated from the subject of interest, without being limited thereto.

In the present invention, the step of dissociating the sample may be performed using collagenase, without being limited thereto.

In the present invention, step (b) of culturing the cells expressing the gene may be performed using a cell culture medium containing fetal bovine serum, a growth factor, and an antibiotic.

In the present invention, step (c) of forming the organoids may be performed using a cell culture medium containing a B27 supplement, a conditioned medium, a growth factor, N-acetylcysteine, and an ALK 5 (TGFβ kinase/activin receptor-like kinase) inhibitor.

The "growth factor" in the present invention is a substance necessary for the growth of cells, and examples thereof include, but are not limited to, vascular endothelial growth factor (VEGF), hepatocyte growth factor (HGF), insulin-like growth factor (IGF), epidermal growth factor (EGF), fibroblast growth factor (FGF), etc.

The "conditioned medium" in the present invention may be at least one selected from the group consisting of Wnt3a conditioned medium, Noggin conditioned medium, and Rspol conditioned medium. For example, the culture medium may contain Wnt3a conditioned medium, Noggin conditioned medium, and Rspo1 conditioned medium, for example, 40% Wnt3a conditioned medium, 10% Noggin conditioned medium, and 10% Rspo1 conditioned medium, without being limited thereto.

The "cell culture medium" in the present invention may contain basic components for the growth and maintenance of the cell line *in vitro,* and may be, for example, Dulbeco's Modified Eagle's Medium (DMEM), Minimal Essential Medium (MEM), Basal Medium Eagle (BME), RPMI-1640, DMEM/F10, DMEM/F12, ADMEM/F12, Glasgow's Minimal essential Medium (GMEM), Iscove's Modified Dulbecco's Medium (IMDM), or the like. The cell culture medium in step (b) may be RPMI-1640, and the cell culture medium in step (c) may be ADMEM/F12, without being limited thereto.

In one embodiment of the present invention, in step (b), the cell culture medium may contain 10% fetal bovine serum, 20 ng/ml epidermal growth factor (EGF), and 1% penicillin-streptomycin, without being limited thereto.

In one embodiment of the present invention, the cell culture medium in step (c) may contain 1.5% B27 supplement, 40% Wnt3a conditioned medium, 10% Noggin conditioned medium, 10% Rspo1 conditioned medium, 50 ng/ml EGF, 100 ng/ml FGF-10, 1.25 mM N-acetylcysteine, and 5 µM A8301 (CAS number: 909910-43-6), without being limited thereto.

The Matrigel in the present invention may be growth factor-reduced Matrigel, without being limited thereto.

### (3) Kidney organoids

Still another embodiment of the present invention provides kidney organoids produced by the method for producing kidney organoids according to the present invention.

The kidney organoids of the present invention are produced using kidney tissue-derived stem cells expressing Lrigl or a gene encoding the same, preferably Lrigl and Klf6 proteins or genes encoding the same, which have excellent stemless. These kidney organoids make it possible to effectively screen a kidney injury therapeutic agent by mimicking the kidney very effectively, and may also be used directly as a cell therapy agent for kidney injury.

In the kidney organoids of the present invention, contents regarding the Lrigl or Klf6 protein or the gene encoding the same, the kidney organoids, and the method for producing the same, etc. are the same as described above in "1. Pharmaceutical composition containing kidney tissue-derived stem cells as an active ingredient" and "(1) Kidney organoids", and thus the description thereof will be omitted.

### (4) Pharmaceutical composition for preventing or treating kidney disease

Another embodiment of the present invention provides a pharmaceutical composition for preventing or treating kidney disease containing kidney organoids as an active ingredient.

The kidney organoids of the present invention may be used as a cell therapy product.

In the pharmaceutical composition for preventing or treating kidney disease according to the present invention, contents regarding the Lrigl protein or the gene encoding the same, the pharmaceutical composition, prevention, treatment, the kidney organoids, the method for producing the same, etc. are the same as described above in "1. Pharmaceutical composition containing kidney tissue-derived stem cells as an active ingredient" and "(1) Kidney organoids", and thus the description thereof will be omitted.

The kidney organoids of the present invention may be administered at any one dose selected from among 1 × 10⁷ to 1 × 10⁸, 1 × 10⁸ to 2 × 10⁸, 2 × 10⁸ to 4 × 10⁸, 4 to 10⁸ to 6 × 10⁸, 6 × 10⁸ to 8 × 10⁸, 8 × 10⁸ to 1 × 10⁹, 1 × 10⁹ to 2 × 10⁹, 2 × 10⁹ to 4 × 10⁹, 4 × 10⁹ to 1 × 10¹⁰, 2 × 10⁸ to 6 × 10⁸, 6 × 10⁸ to 1 × 10⁹, 1 × 10⁸ to 2 × 10⁸, 2 × 10⁸ to 2 × 10⁹, 1 × 10⁷ to 1 × 10⁸, 1 × 10⁸ to 1×10⁹, 1 × 10⁹ to 1 × 10¹⁰, and 1 × 10⁷ to 1 × 10⁹ cells/kg, without being limited thereto.

### 4. Composition, kit and method for detection

### (1) Composition for detecting kidney tissue-derived stem cells

Another embodiment of the present invention provides a composition for detecting kidney tissue-derived stem cells.

The composition for detection according to the present invention contains an agent for measuring the expression level of Lrigl protein or a gene encoding the same.

In addition, the composition for detection according to the present invention may further contain an agent for measuring the expression level of Klf6 protein or a gene encoding the same.

In the composition for detection according to the present invention, contents regarding the Lrigl or Klf6 protein and the kidney tissue-derived stem cells are the same as those described in "1 Pharmaceutical composition containing kidney tissue-derived stem cells as an active ingredient", and thus the description thereof will be omitted.

In the present invention, the agent for measuring the expression level of the gene may be any agent capable of measuring the expression level of DNA present in the biological sample or mRNA transcribed thereby. For example, the agent may be at least one selected from the group consisting of primers, probes, and antisense oligonucleotides, which binds specifically to the gene, without being limited thereto.

In the present invention, the "primer" is a fragment that recognizes a target gene sequence, and comprises a pair of forward and reverse primers, but is preferably a pair of primers providing analysis results with specificity and sensitivity. When the nucleic acid sequence of the primer is a sequence inconsistent with the non-target sequence present in the sample, and thus is a primer that amplifies only the target gene sequence containing the complementary primer binding site without inducing non-specific amplification, high specificity may be imparted.

In the present invention, the "probe" refers to a substance capable of binding specifically to a target substance to be detected in a sample, which is a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, RNA, or DNA, and most preferably PNA. More specifically, the probe is a biomolecule derived from an organism or an analogue thereof, or is one produced *in vitro.* Examples of the probe include an enzyme, a protein, an antibody, a microorganism, an animal and/or plant cell and organ, a neuron, DNA, and RNA. Examples of the DNA include cDNA, genomic DNA, and an oligonucleotide, examples of the RNA include genomic RNA, mRNA and an oligonucleotide, and examples of the protein include antibodies, antigens, enzymes, peptides, and the like.

In the present invention, the "LNA (locked nucleic acid)" refers to a nucleic acid analogue containing a 2'-O or 4'-C methylene bridge. LNA nucleosides comprise the common bases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

In the present invention, the "antisense" means an oligomer that has a nucleotide sequence and a backbone between subunits, wherein an antisense oligomer is hybridized with the target sequence in the RNA by Watson-Crick base pairing to typically allow the formation of the mRNA and RNA:oligomer heterodimers in the target sequence. The oligomer may have an accurate or approximate sequence complementarity to the target sequence.

Since information on the gene of the present invention can be easily identified by a person skilled in the art from the website available through the NCBI accession number, a person skilled in the art can easily construct a primer, probe or antisense nucleotide that binds specifically to the gene, based on the identified gene sequence.

In the present invention, the agent for measuring the expression level of the protein may be any agent capable of measuring the amount of the protein present in the biological sample. For example, the agent may be at least one selected from the group consisting of antibodies, oligopeptides, ligands, peptide nucleic acids (PNAs), and aptamers, which bind specifically to the protein, without being limited thereto.

In the present invention, the "protein" is meant to include not only the protein itself, but also protein isoforms or protein variants that can be produced by splicing and variable promoters or produced due to genetic alterations such as mutations or polymorphisms.

In the present invention, the "antibody" refers to a substance that specifically binds to an antigen and causes an antigen-antibody reaction. For the purposes of the present invention, the antibody refers to an antibody that specifically binds to the biomarker protein. Examples of the antibody of the present invention include all of polyclonal antibodies, monoclonal antibodies, and recombinant antibodies. The antibody may be readily produced using techniques well known in the art. For example, a polyclonal antibody may be produced by a method well known in the art, which includes a process of obtaining a serum containing the antibody by injecting the antigen of the biomarker protein into an animal and collecting blood from the animal. This polyclonal antibody may be produced from any animal such as goat, rabbit, sheep, monkey, horse, pig, cow, dog, or the like. In addition, a monoclonal antibody may be produced using the hybridoma method well known in the art, or the phage antibody library technology. The antibody produced by the above method may be isolated and purified using a method such as gel electrophoresis, dialysis, salt precipitation, ion exchange chromatography, or affinity chromatography. In addition, examples of the antibody of the present invention include not only a complete form having two full-length light chains and two full-length heavy chains, but also functional fragments of an antibody molecule. "Functional fragment of an antibody molecule" refers to a fragment having at least an antigen-binding function, and examples thereof include Fab, F(ab'), F(ab')2 and Fv.

In the present invention, "PNA" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, PNA is used for molecular biology, diagnostic assays and antisense therapies. PNA may be further specified with reference to the background art widely known in the art to which the present invention pertains.

In the present invention, the "aptamer" is an oligonucleotide or a peptide molecule, and general contents regarding the aptamer may be specified with reference to the literature [Bock L C et al., Nature 355(6360):5646(1992); Hoppe-Seyler F, Butz K "Peptide aptamers: powerful new tools for molecular medicine". J Mol Med. 78(8):42630(2000); Cohen B A, Colas P, Brent R. "An artificial cell-cycle inhibitor isolated from a combinatorial library". Proc Natl Acad Sci USA. 95(24): 142727(1998)].

The antibody, oligopeptide, ligand, PNA, aptamer, etc. of the present invention may be easily produced by a person skilled in the art based on the amino acid sequence identifiable from the website available through the NCBI accession number.

### (2) Kit for detecting kidney tissue-derived stem cells

Another embodiment of the present invention provides a kit for detecting kidney tissue-derived stem cells comprising the composition for detection according to the present invention.

In the kit of the present invention, contents regarding the Lrigl protein or the gene encoding the same, the kidney tissue-derived stem cells, the agent for measuring the expression level of the protein, the agent for measuring the expression level of the gene, etc. are the same as described above in "1. "Pharmaceutical composition containing kidney tissue-derived stem cells as an active ingredient" and "(1) Composition for detecting kidney tissue-derived stem cells", and thus the description thereof will be omitted.

The kit of the present invention may be an RT-PCR kit, a DNA chip kit, an ELISA kit, a protein chip kit, a rapid kit, or a multiple reaction monitoring (MRM) kit, without being limited thereto.

The kit of the present invention may further comprise one or more other component compositions, solutions or devices suitable for the assay method.

In one embodiment of the present invention, the kit may further comprise essential elements necessary for carrying out a reverse transcription polymerase reaction. The reverse transcription polymerase reaction kit comprises a pair of primers specific to the gene. The primer is an oligonucleotide having a sequence specific to the nucleic acid sequence of the gene and may have a length of about 7 bp to 50 bp, more preferably about 10 bp to 30 bp. The kit may also comprise a primer specific to the nucleic acid sequence of a control gene (e.g., a housekeeping gene). In addition, the reverse transcription polymerase reaction kit may comprise a test tube or other suitable container, a reaction buffer (various pHs and magnesium concentrations), deoxynucleotides (dNTPs), enzymes such as Taq-polymerase and reverse transcriptase, DNAse, a RNAse inhibitor, DEPC-water, sterilized water, etc., without being limited thereto.

In another embodiment of the present invention, the diagnostic kit of the present invention may comprise essential elements required to perform DNA chip assay. The DNA chip kit may comprise a substrate having immobilized thereon a cDNA or oligonucleotide corresponding to the gene or a fragment thereof, a reagent for constructing a fluorescence-labeled probe, an agent, an enzyme, and the like. In addition, the substrate may comprise a cDNA or oligonucleotide corresponding to a control gene (e.g., a housekeeping gene) or a fragment thereof, without being limited thereto.

In another embodiment of the present invention, the diagnostic kit of the present invention may comprise essential elements required to perform ELISA. The ELISA kit comprise an antibody specific to the protein. The antibody has a high specificity and affinity for the marker protein and exhibits little or no cross-reactivity with other proteins. It is a monoclonal antibody, a polyclonal antibody or a recombinant antibody. The ELISA kit may also comprise an antibody specific to a control protein (e.g., a housekeeping protein). In addition, the ELISA kit may comprise reagents capable of detecting bound antibodies, for example, labelled secondary antibodies, chromophores, enzymes (e.g., conjugated with antibodies), and substrates thereof or other substances capable of binding to antibodies.

### (3) Method for detecting kidney tissue-derived stem cells

Another embodiment of the present invention provides a method for detecting kidney tissue-derived stem cells.

The method for detection according to the present invention comprises a step of measuring the expression level of Lrigl protein or a gene encoding the same from a biological sample isolated from a subject of interest.

In addition, in the present invention, in the step of measuring the expression level, the expression level of Klf6 protein or a gene encoding the same from the biological sample may be further measured.

In the method for detection according to the present invention, contents regarding the kidney tissue-derived stem cells, the Lrigl or Klf6 protein or the gene encoding the same, etc. are the same as described above in "1. Pharmaceutical composition containing kidney tissue-derived stem cells as an active ingredient" and "(1) Composition for detecting kidney tissue-derived stem cells", and thus the description thereof will be omitted.

In the present invention, the "biological sample" refers to any material, biological fluid, tissue or cell obtained or derived from an individual, and may include, for example, blood (including whole blood, leukocytes, peripheral blood mononuclear cells, buffy coat, plasma, and serum), urine, semen, organ secretions, cells, or a cell extract, and may be, for example, kidney tissue or kidney cells, without being limited thereto.

In the present invention, the step of measuring the expression level of the gene may be performed by reverse transcription polymerase reaction (RT-PCR), competitive RT-PCR), real-time RT-PCR, RNase protection assay (RPA), Northern blotting, DNA chip assay, or the like, by using an agent for measuring the expression level of the gene, which is at least one selected from the group consisting of primers, probes, and antisense oligonucleotides, which bind specifically to the gene, without being limited thereto.

In the present invention, the step of measuring the expression level of the protein may be performed by protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA), by using an agent for measuring the expression level of the protein, which is at least one selected from the group consisting of antibodies, oligopeptides, ligands, peptide nucleic acids (PNAs), and aptamers, which bind specifically to the protein, without being limited thereto.

The method for detection according to the present invention may further comprise a step of detecting, as kidney tissue-derived stem cells, cells in which the expression level of the Lrigl protein or the gene encoding the same is higher than a control group.

### 5. Method for isolating kidney tissue-derived stem cells

Another embodiment of the present invention provides a method for isolating kidney tissue-derived stem cells.

The method for isolation according to the present invention comprises a step of isolating cells expressing Lrigl protein or a gene encoding the same from a biological sample isolated from a subject of interest.

In the present invention, the cells in the step of isolating the cells may further express Klf6 protein or a gene encoding the same.

In the method for isolation according to the present invention, contents regarding the Lrigl or Klf6 protein or the gene encoding the same, the kidney tissue-derived stem cells, the biological sample, etc. are the same as described above in "1. Pharmaceutical composition containing kidney tissue-derived stem cells as an active ingredient", "(1) Composition for detecting kidney tissue-derived stem cells" and "(3) Method for detecting kidney tissue-derived stem cells", and thus the description thereof will be omitted.

In the present invention, the step of isolating may be performed by magnetic activated cell sorting (MACS) or flow cytometry analysis.

### 6. Method for culturing kidney tissue-derived stem cells

Another embodiment of the present invention provides a method for culturing kidney tissue-derived stem cells.

The method for culturing according to the present invention comprises steps of: isolating kidney tissue-derived stem cells expressing Lrigl protein or a gene encoding the same; and culturing the isolated kidney tissue-derived stem cells.

In the present invention, the kidney tissue-derived stem cells in the step of isolating may further express Klf6 protein or a gene encoding the same.

In the method for isolation according to the present invention, contents regarding the Lrigl or Klf6 protein or the gene encoding the same, the kidney tissue-derived stem cells, etc. are the same as described above in "1. Pharmaceutical composition containing kidney tissue-derived stem cells as an active ingredient", and thus the description thereof will be omitted.

The method for culturing according to the present invention may comprise isolating kidney tissue-derived stem cells according to the method described in "5. Method for isolating kidney tissue-derived stem cells" herein, and then culturing the same, for example, *in vitro,* without being limited thereto.

"Culturing" in the step of culturing according to the present invention means any *in vitro* culturing of cells. Culturing in the present invention may be performed using a cell culture medium, and the cell culture medium refers to any type of medium that is used in a cell culture environment, and may contain, for example, amino acids, at least one carbohydrate as an energy source, trace elements, vitamins, salts, and possible additional ingredients (e.g., to affect cell growth, productivity, or product quality), without being not limited thereto.

### 7. Method for producing animal model

Another embodiment of the present invention provides a method for producing an animal model for screening a cell therapy product for preventing or treating kidney disease.

The method for producing an animal model according to the present invention comprises steps of: inducing kidney injury in an animal in which a gene of interest is conditionally expressed by a CreERT2-LoxP system; and inducing expression of the gene of interest in the animal by treatment with an estrogen antagonist.

In the method for producing an animal model according to the present invention, contents regarding the kidney tissue-derived stem cells, the kidney disease, and the cell therapy product are the same as described in "1. Pharmaceutical composition containing kidney tissue-derived stem cells as an active ingredient", and thus the description thereof will be omitted.

In the present invention, the "CreERT2-LoxP system" refers to a system in which a promoter-specifically expressed Cre-ERT2 polypeptide recognizes two LoxP nucleotide sequences and catalyzes site-specific recombination between the two LoxP nucleotide sequences, thereby specifically expressing a gene downstream of the LoxP nucleotide sequences. A method of constructing this system may be easily performed by a person skilled in the art according to the contents described in Lab Anim Res. Dec 2018; 34(4): 147-159.

In the present invention, the "gene of interest" refers to a gene that is specifically expressed or expected to be expressed in cells that have the potential to be used as a cell therapy product. For the purpose of the present invention, the "gene of interest" may be a gene encoding Lrig1, preferably a gene encoding Lrigl and a gene encoding Klf6, without being limited thereto.

In the present invention, the "animal model" refers to an animal that can exhibit a form of disease that is very similar to the human disease or the like. When this animal model is used, it is possible to study the cause and pathogenesis of various diseases, and to obtain basic data for determining possibilities such as selection of a therapeutic agent or performance of a toxicity test.

The term "animal" in the present invention refers to any non-human mammals, and includes animals of all ages, including embryos, fetuses, newborns, and adults. This animal may be any one selected from the group consisting of rabbits, rodents (e.g., mice, rats, hamsters, gerbils or guinea pigs), cattle, sheep, pigs, goats, horses, dogs, cats, birds (e.g., chickens, turkeys, ducks, or geese), and primates (e.g., chimpanzees, monkeys, or rhesus monkeys), without being limited thereto.

In the present invention, the step of inducing kidney injury may be performed by any one selected from the group consisting of intraperitoneal administration of folic acid, induction of ischemia/reperfusion injury, and induction of unilateral ureteral obstruction, without being limited thereto.

In the present invention, the folic acid may be intraperitoneally administered at a dose of 100 mg/kg to 300 mg/kg, for example, 150 mg/kg or 250 mg/kg, without being limited thereto. If the dose of folic acid is less than 100 mg/kg, acute kidney injury may not be induced in the kidney, and if the dose is more than 300 mg/kg, it may cause toxicity in the animal model, affecting the interpretation of the desired experimental results or resulting in death of the animal model.

The estrogen antagonist in the present invention may be tamoxifen, 4-hydroxytamoxifen, clomifen, raloxifene, or a combination thereof, without being limited thereto.

### 8. Animal model

Another embodiment of the present invention provides an animal model for screening a cell therapy product for preventing or treating kidney disease, produced by the production method of the present invention.

In the animal model of the present invention, contents regarding the production method, the cell therapy product, the kidney disease, etc. are the same as described above in "1. Pharmaceutical composition containing kidney tissue-derived stem cells as an active ingredient" and "6. Method for producing animal model, and thus the description thereof will be omitted.

### Advantageous Effects

The kidney tissue-derived stem cells or organoids according to the present invention are easy to apply for treatment, may be supplied in large amounts due to their high self-renewal capacity, have an excellent ability to differentiate into kidney cells, are less likely to form tumors, and have an excellent ability to regenerate damaged tissue when injected directly into lesions. Therefore, they may be used very suitably for regenerative therapy based on, in particular, adult stem cells among these stem cells.

In addition, the composition according to the present invention is capable of specifically selecting only kidney tissue-derived stem cells among kidney cells. Furthermore, kidney tissue-derived stem cells expressing Lrigl protein or a gene encoding the same have excellent self-renewal and pluripotent abilities and are able to differentiate into nephrons, and thus they may be used very effectively for the prevention or treatment of kidney disease.

### Brief Description of Drawings

FIG. 1 is a schematic view showing an experimental process that is performed using a mouse animal model in an *in vivo* lineage tracing study according to an example of the present invention.
FIG. 2 is a schematic view showing an experimental process that is performed using an embryonic mouse animal model in an *in vivo* lineage tracing study according to an example of the present invention.
FIG. 3 is a schematic view showing a method for producing an animal model of high-dose (250 mg/kg) folic acid-induced acute kidney injury according to an example of the present invention.
FIG. 4 is a schematic view showing a process of transplanting kidney organoids of the present invention into an animal model of high-dose (150 mg/kg) folic acid-induced acute kidney injury and then performing analysis, according to an example of the present invention.
FIG. 5 is a schematic view showing a method for producing an animal model of ischemia/reperfusion injury-induced acute kidney injury according to an example of the present invention.
FIG. 6 is a schematic view showing a method for producing an animal model of unilateral ureteral obstruction-induced acute kidney injury according to an example of the present invention.
FIG. 7 shows the results of confirming the presence or absence of tdTomato+ cells (LrigltdT+) in the mouse kidney by tissue staining according to an example of the present invention.
FIG. 8 shows the results of quantifying the number of tdTomato+ cells (LrigltdT+) in the mouse kidney according to an example of the present invention.
FIG. 9 shows the results of confirming the presence or absence of tdTomato+ cells (LrigltdT+) in a mouse at the developmental stage by tissue staining according to an example of the present invention.
FIG. 10 shows the results of quantifying the number of tdTomato+ cells (LrigltdT+) in a mouse at the developmental stage according to an example of the present invention.
FIG. 11 shows the results of observing a kidney organoid using a fluorescence microscope according to an example of the present invention.
FIG. 12 shows the result of analyzing the expression levels of genes expressed in kidney organoids by real-time polymerase chain reaction according to an example of the present invention.
FIG. 13 shows results indicating that the number of kidney organoids increased when kidney organoids were cultured for a long period of time from day 1 to day 19 according to an example of the present invention.
FIG. 14 shows the results of performing tissue staining and analysis of the expression level of KIM1 to confirm kidney repair after transplanting PBS or the kidney organoids of the present invention into an animal model of high-dose (150 mg/kg) folic acid-induced acute kidney injury, according to an example of the present invention.
FIG. 15 shows the results of analyzing blood BUN and creatinine concentrations to confirm kidney repair after transplanting PBS or kidney organoids of the present invention into an animal model of high-dose (150 mg/kg) folic acid-induced acute kidney injury, according to an example of the present invention.
FIG. 16 shows the result of analyzing the expression of Ki-67 in kidney organoids by fluorescence microscopy according to an example of the present invention.
FIG. 17 shows the results of classifying proximal tubule (PT) clusters in kidney organoids into four sub-clusters (PTS1, PTS2, PTS3, and PTQPs) according to an example of the present invention.
FIGS. 18 and 19 are bubble plots showing the results of analyzing the expression levels of genes in each of PTS1, PTS2, PTS3 and PTQPs clusters in kidney organoids according to an example of the present invention.
FIG. 20 shows the results of measuring the percentages of PTS1, PTS2, PTS3, and PTQPs cluster cells on day 1 and day 365 after Cre-loxp induction following transplantation of kidney organoids according to an example of the present invention.
FIG. 21 shows the results of measuring changes in the numbers of PTS1, PTS2, PTS3, and PTQPs cluster cells on day 365 compared to day 1 after Cre-loxp induction following transplantation of kidney organoids according to an example of the present invention.
FIG. 22 depicts violin plots showing the results of analyzing the expression levels of self-renewal-related genes, quiescence-related genes, and pluripotent/immature cell-related genes in each of PTS1, PTS2, PTS3 and PTQPs clusters in kidney organoids according to an example of the present invention.
FIG. 23 shows the top 10 genes highly expressed in each of PTS3 and PTQPs clusters in kidney organoids according to an example of the present invention.
FIG. 24 shows immunofluorescence staining images of LTL (PT marker) and KLF6 in kidney sections on day 1 and day 365 after Cre-loxp induction following transplantation of kidney organoids according to an example of the present invention.
FIG. 25 shows the results of quantifying LTL+KLF6+ cells per 20X field in kidney sections on day 1 and day 365 after Cre-loxp induction following transplantation of kidney organoids according to an example of the present invention (N=3; 24 images).
FIG. 26 shows the percentage of tdTomato+ cells in each cluster on day 1 and day 365 after Cre-loxp induction following transplantation of kidney organoids according to an example of the present invention.
FIG. 27 shows the results of classifying the pseudotime lineage in the proximal tubules (PT) of kidney organoids into three different states according to an example of the present invention. Here, the PTS1, PTS3, and PTQPs clusters are indicated by different colors, and the black arrows indicate the direction of pseudotime.
FIG. 28 shows the results of plotting tdTomato+ cells on a phylogenetic graph on day 1 and day 365 after Cre-loxp induction following transplantation of kidney organoids according to an embodiment of the present invention, and plotting a heat map representing color-coded expression levels. Here, low expression is shown in gray, and high expression is shown in red.
FIG. 29 shows immunofluorescence staining images of LrigltdTomato+ and KLF6 staining in kidney sections on day 1 and day 365 after Cre-loxp induction following transplantation of kidney organoids according to an example of the present invention.
FIG. 30 shows the results of quantifying LrigltdTomato + KLF6 + cells per 20X field in kidney sections on day 1 and day 365 after Cre-loxp induction following transplantation of kidney organoids according to an example of the present invention (N=5; 42 images).

### Best Mode

The present invention is intended to overcome the limitations of conventional treatment of kidney disease such as acute renal failure, and to develop a more effective therapeutic agent. The present invention is intended to develop an effective therapeutic agent for kidney disease using kidney tissue-derived stem cells or organoids. In the case of treatment only with high-concentration folic acid (FA) and the case of treatment with high-concentration folic acid and PBS injection (FA + PBS), blood BUN and creatinine levels were very high, whereas, when kidney organoids were injected, blood BUN and creatinine levels were reduced to levels similar to normal levels. These results directly indicate that Lrigl-positive kidney organoids may be used very effectively for the treatment of damaged kidney.

### Mode for Invention

### [Experimental Methods]

### [Experimental Method 1] Experimental animals

All *in vivo* experiments conducted in the present specification were approved by the Yonsei University Institutional Animal Care and Use Committee (IACUC 2017-0325).

Experimental animals were housed in a specific pathogen-free (SPF) barrier facility under 12-hour alternating light-dark cycles, and raised by feeding PicoLab Lab Rodent Diet 20 (LabDiet, St. Louis, MO, USA).

For experimental animals used in *in vivo* experiments, 1) Lrig1CreERT2/+ were provided by Robert J. Coffey at Vanderbilt University, 2) B6.Cg-Gt(ROSA)26Sortm14(CAG-tdTomato)/Hze/J (R26R-LSL-tdTomato; The Jackson Laboratory, 007914) were provided by Professor Bok Jin-Woong at Yonsei University, and 3) B6.129(Cg)-Gt(ROSA)26Sortm4(ACTB-tdToamto-EGFP)Luo/J (ACTB-mT/mG; The Jackson Laboratory, 007676) were provided by Professor Hyun-Woong Ki at Yonsei University.

### [Experimental Method 2] Methods for in vivo lineage tracing studies

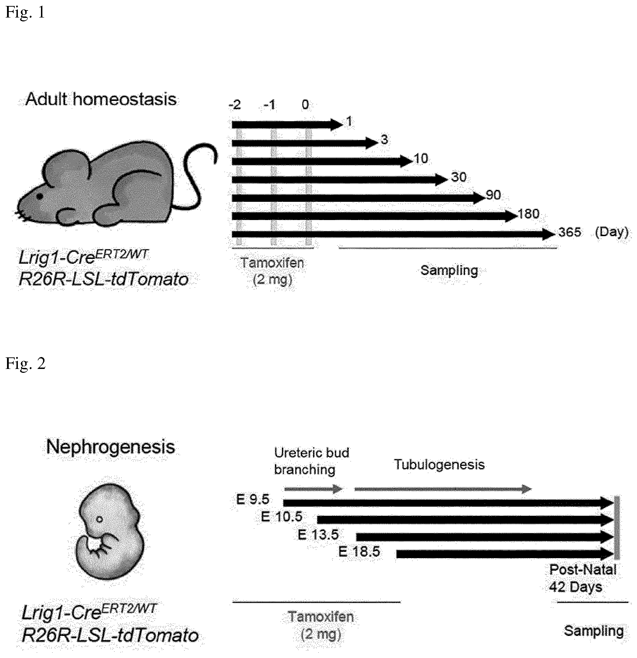

As shown in FIG. 1, heterozygous mice were produced by mating Lrig1CreERT2/+ mice and R26R-LSL-tdTomato mice, which are homozygous reporter mice described in Experimental Method 1 above.

In addition, as shown in FIG. 2, 2 mg of 4-hydroxytamoxifen (Sigma-Aldrich) contained in corn oil was injected once into E9.5, E10.5, E13.5 and E18.5 embryos generated by mating Lrig1CreERT2/+ mice and R26R-LSL-tdTomato mice, which are homozygous reporter mice, and analysis was performed at post-natal 6 weeks.

### [Experimental Method 3] In vitro 2D and organoid culture method

For 2D and organoid culture, homozygous reporter mice produced by mating Lrig1CreERT2/+ mice and R26R-ACTB-mT/mG mice were bred for 6 to 10 weeks, and then primary kidney epithelial cells were collected therefrom. 2 mg/ml of type 1 collagenase was added to the collected primary kidney epithelial cells and cultured at 37°C for 30 minutes with gentle agitation. Thereafter, the primary kidney epithelial cells were filtered through a filter, and then the isolated single cells were cultured. Next, cells expressing Lrigl protein were added to RPMI 1640 medium (containing 10% fetal bovine serum (FBS), 20 ng/ml EGF, and 1% penicillin-streptomycin), and cultured to a confluence of about 80% for 7 to 8 days at 37°C under 5% CO₂. Thereafter, the cultured cells were dispensed into wells containing growth factor-reduced Matrigel and culture medium at a density of 1 × 10³ cells/well and cultured. The culture medium used here was based on ADMEM/F12 culture medium containing 1% penicillin-streptomycin, HEPS, and Glutamax, and contained 1.5% B27 supplement, 40% Wnt3a conditioned medium (produced using stably transfected L cells), 10% Noggin conditioned medium, 10% Rspo1 conditioned medium, 50 ng/ml EGF, 100 ng/mL FGF-10, 1.25 mM N-acetylcysteine, and 5 µM A8301 (CAS Number 909910-43-6).

After the cells were sufficiently polymerized in Matrigel, an organoid culture medium was added, and the organoid culture medium was replaced every 3 days.

### [Experimental Method 4] Construction of acute kidney injury animal model

### [4-1] Construction of animal model of high-dose folic acid-induced acute kidney injury

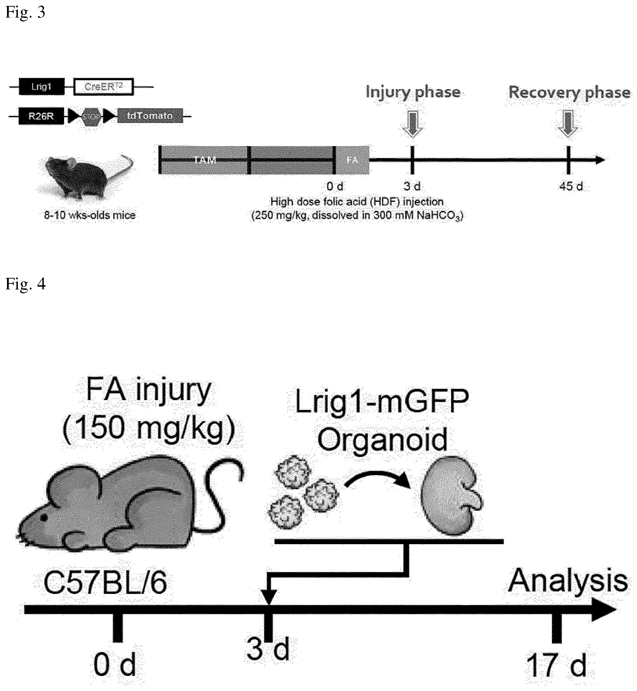

As shown in FIG. 3, tamoxifen was injected into adult (8 to 10 weeks old) Lrigl-CreERT2;LSL-tdTomato mice as described in Experimental Method 2 so that Lrigl protein could be expressed. Then, 250 mg/kg of folic acid (FA) was intraperitoneally injected to induce acute renal injury.

In addition, as shown in FIG. 4, 150 mg/kg of folic acid was intraperitoneally injected into C57BL/6 mice to induce acute renal injury, and on day 3 after folic acid injection, the kidney organoids produced in Experimental Method 3 were transplanted orthotopically. Specifically, C57BL/6 mice (n=3) with acute renal injury induced were anesthetized using isoflurane, and the kidneys were exposed to the outside by incising the flanks. Then, PBS as a control or about 40 kidney organoids produced in Experimental Method 3 above were injected directly into the cortical region of the kidney at least 15 times. Then, the mice were bred for 11 days for analysis. Then, on day 14, blood was sampled from the mice to measure plasma creatinine and BUN levels.

### [4-2] Construction of animal model of ischemia/reperfusion injury-induced acute kidney injury

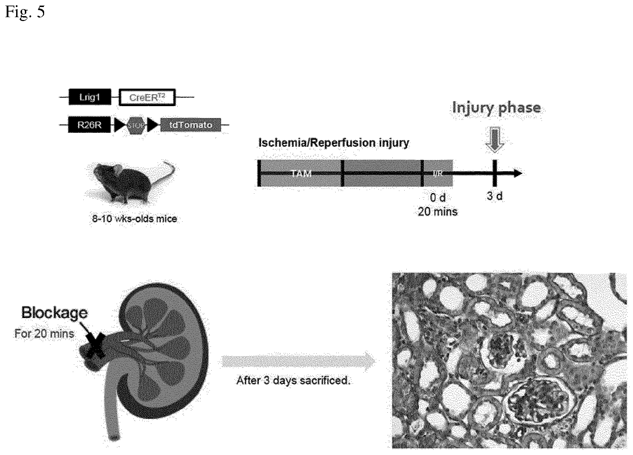

As shown in FIG. 5, tamoxifen was injected into adult (8 to 10 weeks old) Lrigl-CreERT2;LSL-tdTOmato mice as described in Experimental Method 2 so that Lrigl protein could be expressed. Then, acute renal injury was induced by occluding the mouse renal artery with forceps for 20 minutes. After 3 days, as a result of collecting and analyzing the kidney tissues from the mice, it could be confirmed that acute kidney injury was induced.

### [4-3] Construction of animal model of unilateral ureteral obstruction-induced acute kidney injury

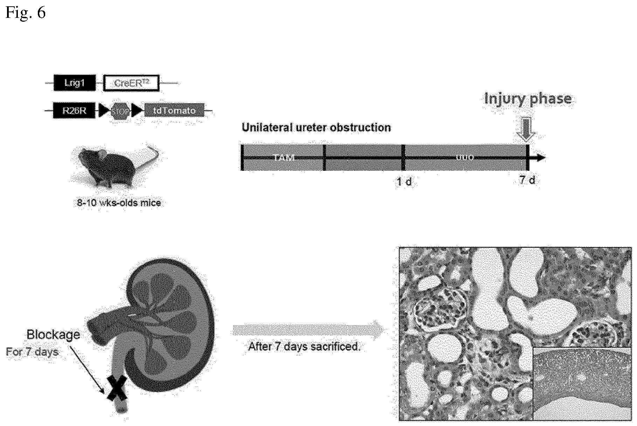

As shown in FIG. 6, tamoxifen was injected into adult (8 to 10 weeks old) Lrigl-CreERT2;LSL-tdTomato mice as described in Experimental Method 2 so that Lrigl protein could be expressed. Then, acute renal injury was induced by occluding the ureter with forceps for 7 days. After 7 days, as a result of collecting and analyzing the kidney tissues from the mice, it was confirmed that acute kidney injury was induced.

### [Experimental Results]

### [Experimental Results 1] Pedigree analysis by tamoxifen induction of Lrig1-tdTomato progeny in mouse kidney

To analyze the behavior of cells expressing Lrigl in the kidney and their progeny, lineage tracing analysis was performed using the R26R-LSL-tdTomato mouse model.

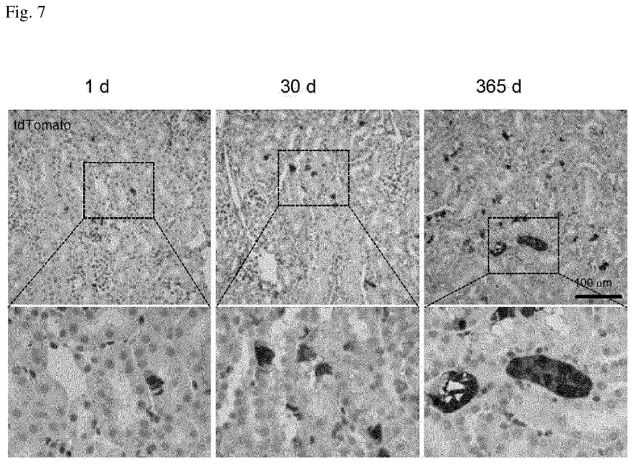

As shown in FIG. 7, it was confirmed that, on day 1, a very few level of tdTomato+ cells were observed in the cortex, whereas on day 365, progeny cells induced by Lrigl-expressing cells formed tubular structures in 12 (±2.4)% of the whole kidney.

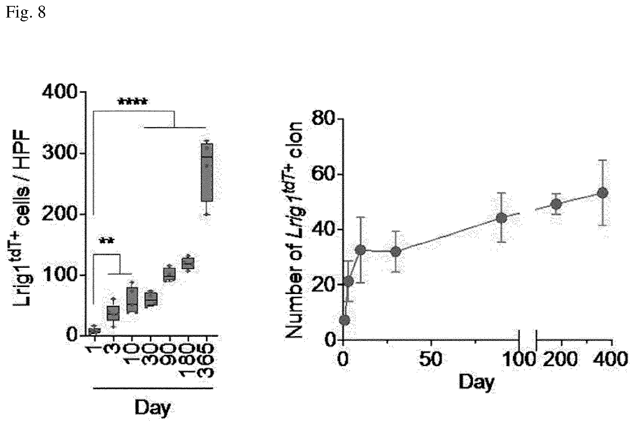

As shown in FIG. 8, when the number of Lrigl-expressing cells (LrigltdT+) was quantified as a function of time (days), it was confirmed that these cell clones gradually increased with the passage of time, and this increase lasted up to 365 days after Lrigl expression.

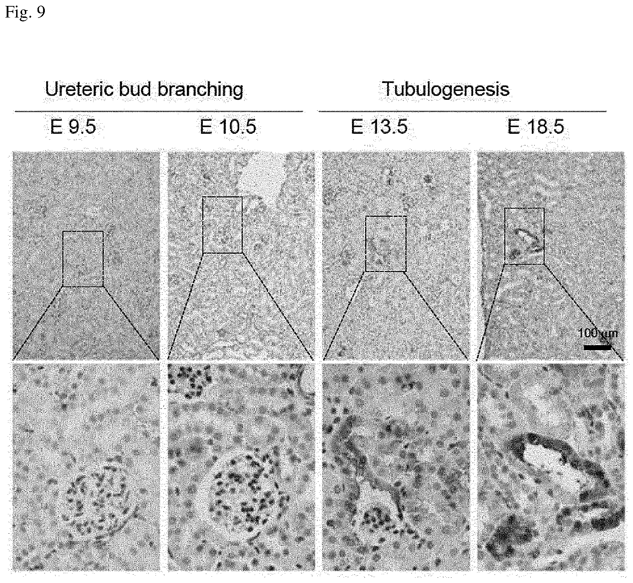

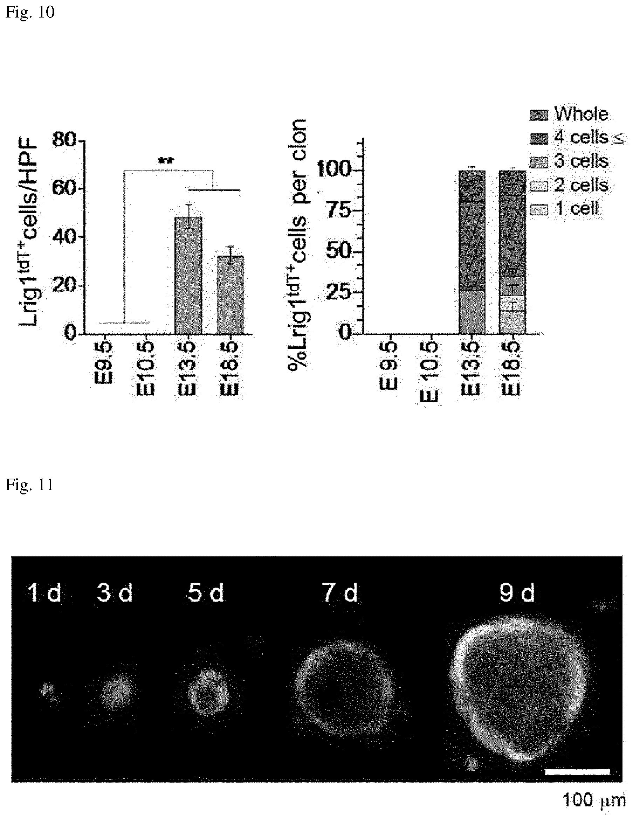

As shown in FIGS. 9 and 10, tdTomato+ cells (LrigltdT+) were not observed at the ureteric bud branching stage (E9.5 and E10.5). However, tdTomato+ cells were observed at E13.5, which is the nephrogenesis stage, and most of the cells were expanded to form tubular structures.

From these results, it can be seen that Lrig1-expresing cells are a stem cell population which is involved in nephron differentiation after initial development into a mature kidney.

### [Experimental Results 2] Confirmation of therapeutic effect by orthotopic transplantation of kidney organoids in animal model of acute kidney injury induced by high-concentration folic acid

As shown in FIG. 11, as a result of analyzing the kidney organoids, produced by the method described in Experimental Method 3, using a fluorescence microscope, a number of green Lrigl cells were observed on days 7 and 9 after culturing the kidney organoids.

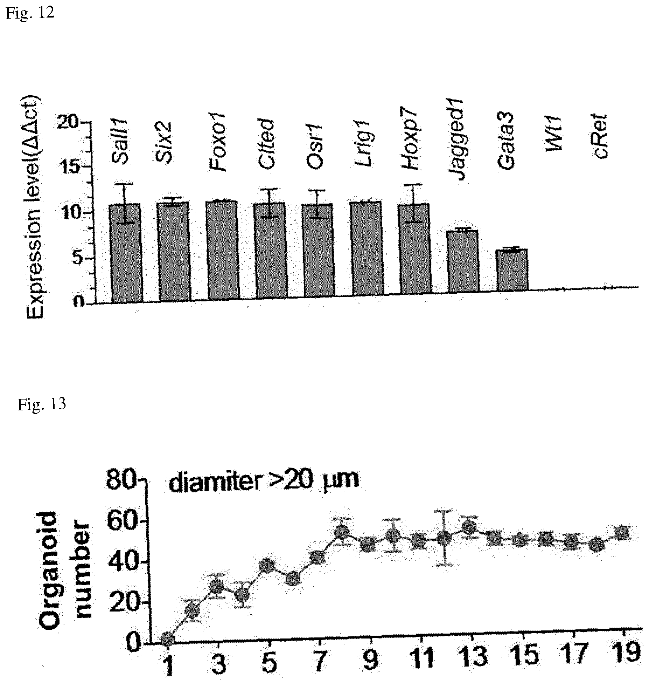

In addition, as shown in FIG. 12, as a result of analyzing the expression levels of genes by Real-time PCR using the primers shown in Table 1 below according to a conventional method on day 17 after culturing the kidney organoids, it was confirmed that not only the expression level of Lrigl but also the expression levels of kidney stem cell genes such as Sall1, Six2, Foxo1, Cited, Osr1, Hoxp7, Jagged1 and Gata3 increased. Furthermore, as shown in FIG. 13, in the case of Lrigl-positive kidney organoids, the number of organoids produced increased to about 40 or more from day 9 to day 19.

**[Table 1]**

| SEQ ID NO. | Gene | Characteristic | Nucleotide sequence |
|---|---|---|---|
| SEQ ID NO: 3 | mLrig1 | Forward | GGTGAGCCTGGCCTTATGTGAATA |
| SEQ ID NO: 4 | | Reverse | CACCACCATCCTGCACCTCC |
| SEQ ID NO: 5 | Osrl | Forward | TACTCTTTCCTTCAGGCAGTGA |
| SEQ ID NO: 6 | | Reverse | GATCGAGGCAAGTGCATGG |
| SEQ ID NO: 7 | Six2 | Forward | CACCTCCACAAGAATGAAAGCG |
| SEQ ID NO: 8 | | Reverse | CTCCGCCTCGATGTAGTGC |
| SEQ ID NO: 9 | 1 | Forward | AACCTTGGAGTGAAGGATCGC |
| SEQ ID NO: 10 | | Reverse | GTAGGAGAGCCTATTGGAGATGT |
| SEQ ID NO: 11 | Salll | Forward | CTCAACATTTCCAATCCGACCC |
| SEQ ID NO: 12 | | Reverse | GGCATCCTTGCTCTTAGTGGG |
| SEQ ID NO: 13 | WT1 | Forward | GAGAGCCAGCCTACCATCC |
| SEQ ID NO: 14 | | Reverse | GGGTCCTCGTGTTTGAAGGAA |
| SEQ ID NO: 15 | Hoxb1 | Forward | AAGTTCGGTTTTCGCTCCAGG |
| SEQ ID NO: 16 | | Reverse | ACACCCCGGAGAGGTTCTG |
| SEQ ID NO: 17 | Gata3 | Forward | CTCGGCCATTCGTACATGGAA |
| SEQ ID NO: 18 | | Reverse | GGATACCTCTGCACCGTAGC |
| SEQ ID NO: 19 | cRet | Forward | GCGTCAGGGAGATGGTAAAG |
| SEQ ID NO: 20 | | Reverse | CATCAGGGAAACAGTTGCAG |
| SEQ ID NO: 21 | Foxdl | Forward | CGCTAAGAATCCGCTGGTGAAG |
| SEQ ID NO: 22 | | Reverse | GGATCTTGACGAAGCAGTCGTT |
| SEQ ID NO: 23 | Jagged1 | Forward | CCTCGGGTCAGTTTGAGCTG |
| SEQ ID NO: 24 | | Reverse | CCTTGAGGCACACTTTGAAGTA |

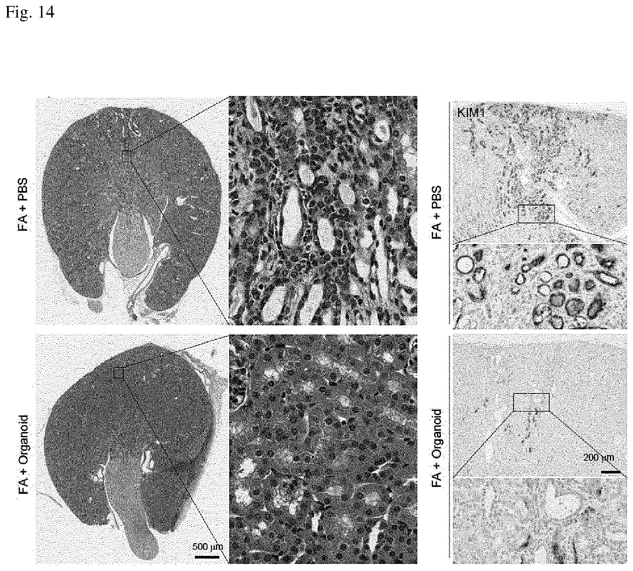

As shown in FIG. 14, PBS (FA + PBS) or kidney organoids (FA + Organoid) was injected, according to the method of Experimental Method 4, directly into the kidney of the animal model of acute kidney injury induced by high-concentration folic acid, described in [4-1] of Experimental Method 4 above, and after 14 days, H&E staining was performed according to a conventional method. As a result, it was confirmed that, when only PBS was injected (FA + PBS), the kidney injured by high-concentration folic acid was not repaired and the expression level of KIM1 was high, whereas, when the kidney organoids were injected, the kidney injured by high-concentration folic acid was repaired and the expression level of KIM1 was reduced. From these results, it can be seen that injection of the kidney organoids can repair injured kidneys very effectively.

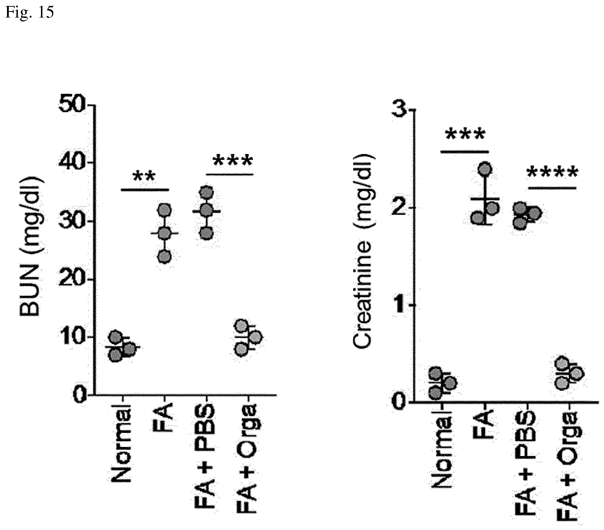

In addition, as shown in FIG. 15, when the kidney was treated only with high-concentration folic acid (FA) or and when the kidney was treated with high-concentration folic acid and injected with PBS (FA + PBS), blood BUN and creatinine levels were very high, whereas when the kidney organoids were injected, blood BUN and creatinine levels were reduced to levels similar to normal levels. These results directly indicate that Lrigl-positive kidney organoids may be used very effectively for the treatment of damaged kidneys.

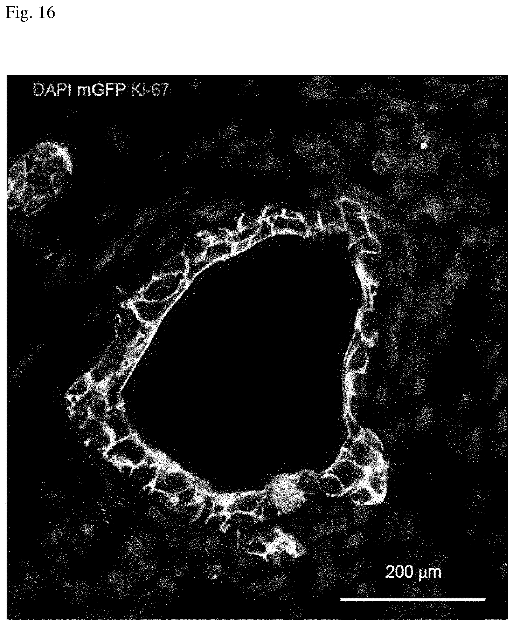

In addition, as shown in FIG. 16, as a result of observation through a fluorescence microscope, organoids injected into the kidney were confirmed immunofluorescence staining, and it could be seen that proliferation markers such as Ki-67 were expressed, indicating that the organoids injected into the kidney were proliferative.

### [Experimental Results 3] Effects of Lrigl-positive cells and their descendants on stem cell niche formation in adult kidney

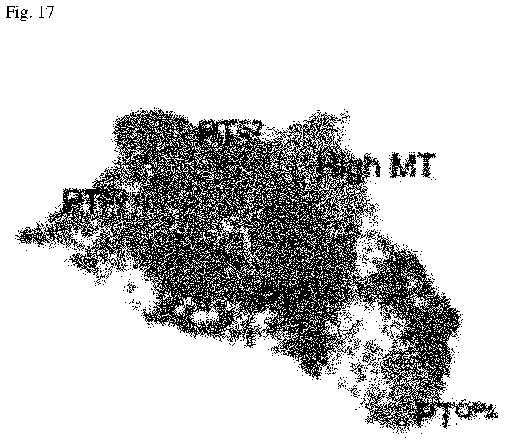

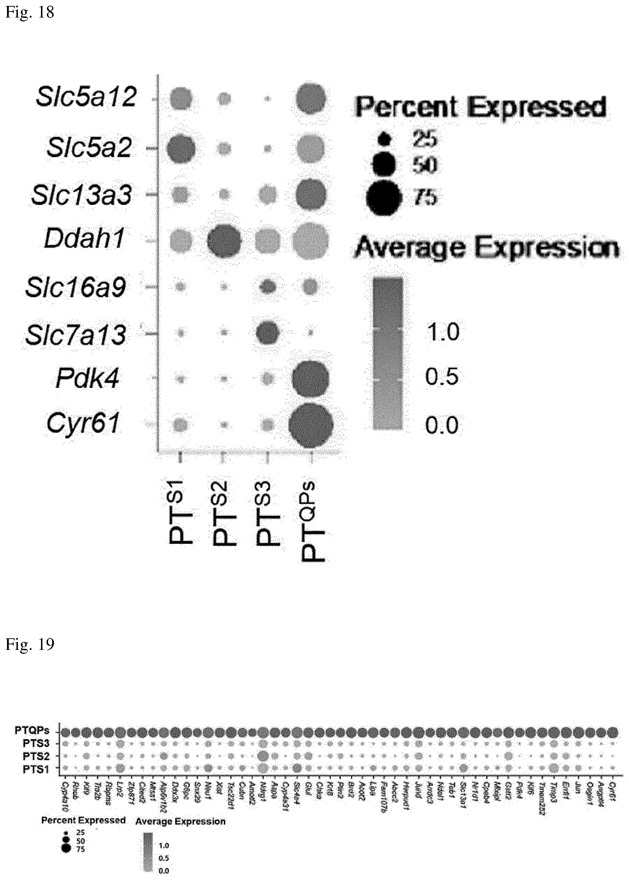

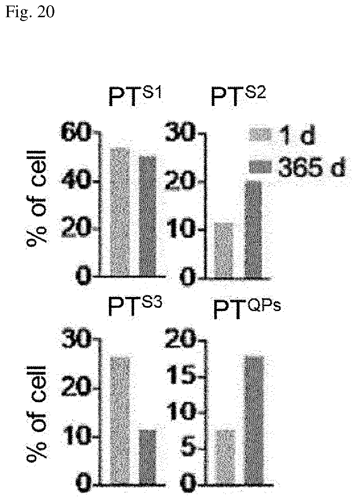

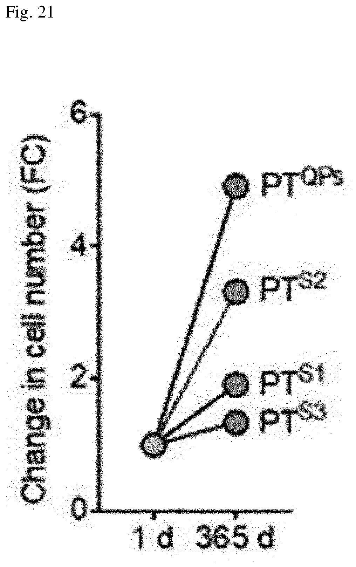

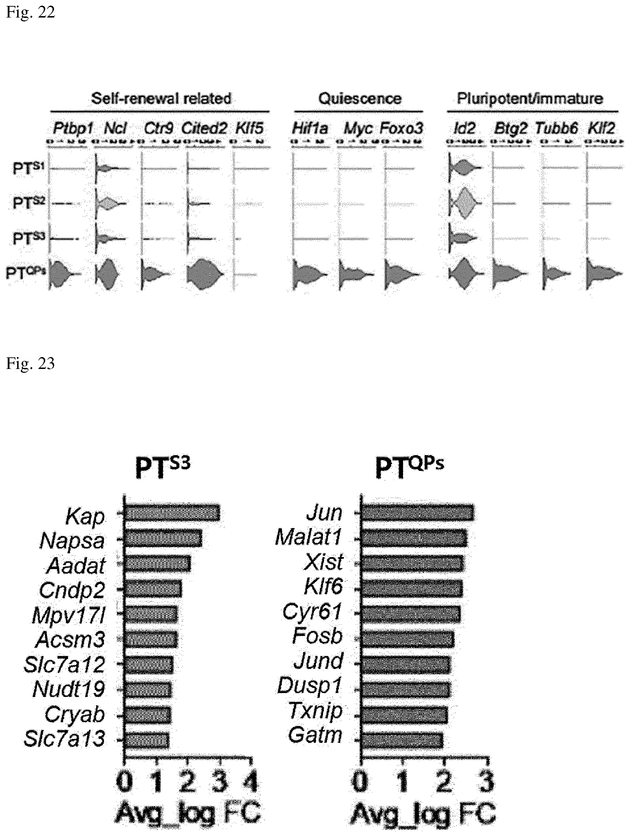

From the previous experimental results, it could be seen that Lrigl-positive cells survive for a long time in the proximal tubule (PT) and correspond to potential kidney stem/progenitor cells, and descendants from the Lrigl-positive cells substantially contributes to maintaining PT homeostasis. In order to confirm the cellular heterogeneity of Lrig1+ cells and descendants therefrom in PT, as shown in FIG. 17, the PT cluster was classified into four sub-clusters: PTS1, PTS2, PTS3, and PTQPs. At this time, Slc5a12 and Slc5a2 markers were used for PTS1 classification, Slc13a3 and Ddah1 markers for PTS2 classification, and Slc16a9 and Slc7a13 markers for PTS3 classification. In addition, as shown in FIG. 18, the present inventors found the sub-cluster PTQPs which abundantly express pyruvate dehydrogenase kidney 4 (Pdk4) and cysteine-rich protein 61 (Cyr61), which are up-regulated in acute kidney injury conditions. For more specific definition, PTQPs was compared to the other PT sub-clusters. As a result of analyzing the expression level of the top 50 genes expressed in PTQPs, as shown in FIG. 19, it could be confirmed that genes related to kidney injury and repair were highly expressed in PTQPs, and that two nephron progenitor genes were also expressed therein. As a result of measuring the cell number in each PT sub-cluster, as shown in FIG. 20, it was confirmed that the number of PTS2 and PTQPs cells increased on day 365 after Cre-loxp recombination. In addition, as shown in FIG. 21, it could be confirmed that the number of PTQPs sub-cluster cells in the kidney on day 365 after Cre-loxp recombination was 5 times larger than that in the kidney on day 1. This suggests that the number of PT cells increased over time, and in particular, the number of PTQPs and PTS2 cluster cells increased. Next, the present inventors defined the PTQPs sub-cluster in the kidney using the adult stem cell gene module. As a result, a total of 650 genes were detected in DEGs. As shown in FIG. 22, it could be confirmed that stemness-related genes were highly expressed in the PTQPs cluster, and self-renewal-related genes (Ptbp1, Ncl, Ctr9 and Cited2, excluding Klf5), quiescence-related genes (Hifla, Myc, and Foxo3), and pluripotent or immature cell-related genes (Id2, Btg2, Tubb6, and Klf2) were upregulated. This suggests that the PTQPs sub-cluster expresses stemness-related genes, including genes related to kidney injury repair and markers of mature PT cells. Based on these results, the corresponding sub-cluster was named "PT quiescent progenitors (PTQPs)".

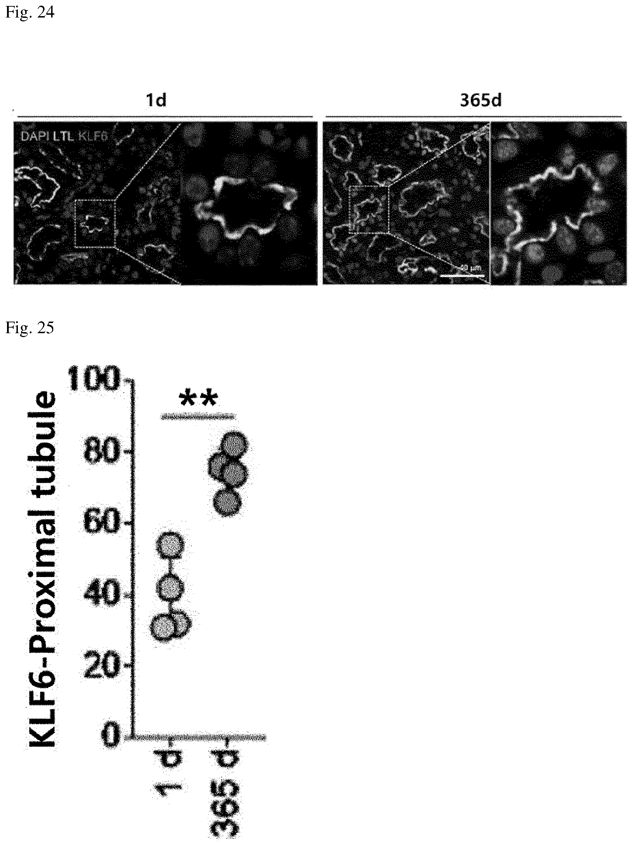

Next, as a result of identifying markers of PTQPs distinct from PTS3, as shown in FIG. 23, genes known as stem cell niches that are highly expressed in DEGs could be identified. PT segment 3-specific genes were highly expressed in the PTS3 sub-cluster, whereas they were not highly expressed in PTQPs. To verify PTQPs markers, kidney sections were IF stained using Jun, Klf6 and Cyr61. Jun and Cyr61 were excluded because they were highly expressed not only in PT, but also in various nephron segments. Next, KLF6 and LTL stained in kidney sections on day 1 and day 365 were stained in order to confirm whether they would be expressed. As shown in FIG. 24, Klf6+LTL+ PT tubules were weakly expressed in kidney sections on day 1, whereas Klf6+LTL+ PT tubules were strongly expressed in kidney sections on day 365. As a result of quantification, as shown in FIG. 25, it could be confirmed that KLF6+ expressing PT tubules significantly increased in the kidney on day 365. This suggests that an increased distribution of PTQPs in the kidney on day 365 can be confirmed using Klf6.

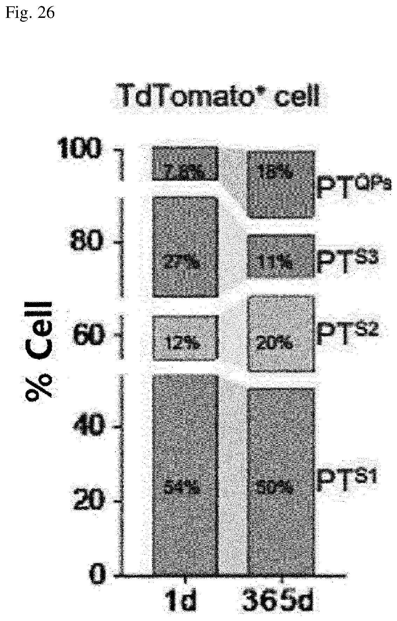

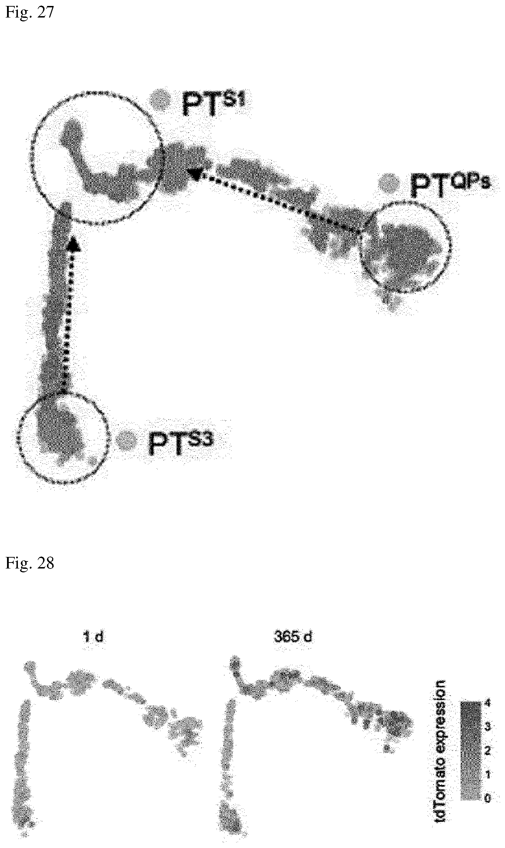

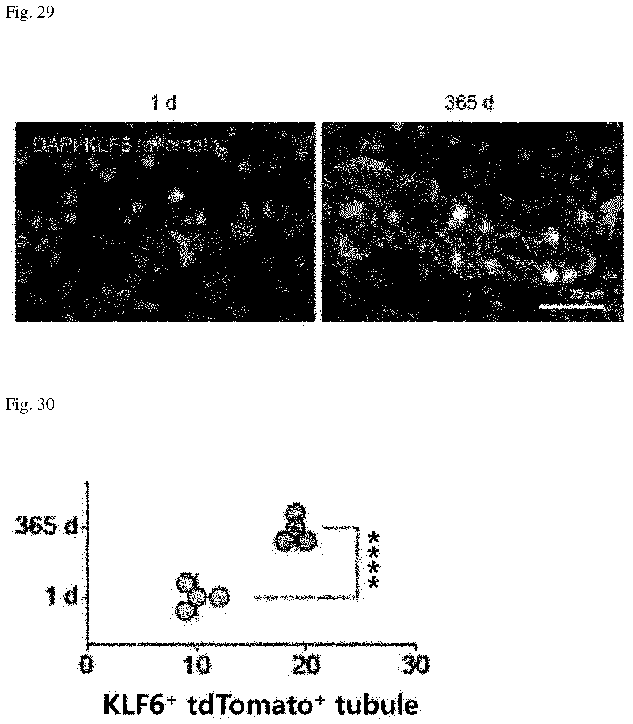

To confirm whether descendants from Lrigl-positive cells form the PTQPs population, the present inventors checked tdTomato-expressing cells in kidney sections on day 1 and day 365. As a result, as shown in FIG. 26, tdTomato+ cells accounted for 54% of PTS1 cells and 27% of PTS3 cells in the kidney section on day 1, which, however, decreased to 50% and 11% in the kidney section on day 365, respectively. On the other hand, tdTomato+ cells accounted for 12% of PTS2 cells and 17.8% of PTQPs cells in the kidney section on day 1, which, however, increased to 20% and 18% on day 365, respectively. This suggests that descendants from Lrigl-positive cells form the PTQPs population. In addition, cell trajectory showing progression toward differentiation was analyzed through information on single cells. The initial stage of the trajectory corresponded to PTS3 known as PT stem cell niche and PTQPs identified as new stem cell niche. This cell population can be divided into two distinct trajectories to PTS1 (FIG. 27). It could be seen that, in the kidney on day 1, PTS3 was dominant and then differentiated to the PTS1 sub-cluster, and on day 365, the PTQPs cluster mainly performed mainly the cellular homeostasis of the PTS1 sub-cluster. As a result of aligning tdTomato-expressing cells with PTS3 and PTQPs on day 1 and day 365, as shown in FIG. 28, it could be confirmed that, on day 1, tdTomato-expressing cells in the PTS3 cluster differentiated into PTS1, and on day 365, tdTomato-expressing cells in the PTQPs cluster mainly maintained PTS1. As a result of staining Klf6 and tdTomato+ cells, as shown in FIGS. 29 and 30, it could be confirmed that Klf6+tdTomato+ tubules significantly increased on day 365 compared to day 1. This suggests that Lrigl-positive cells and their descendants form the PTQPs cluster. Even in the experimental conditions, kidneys on day 1 were taken from 6-week-old mice, while kidneys on day 365 were taken from 13-month-old mice. From the above experimental results, it could be seen found that kidney homeostasis was regulated by PTS3 in the young kidney (kidney on day), and PTQPs showed a new stem cell niche in the aged kidney (kidney on day 365).

Although the present invention has been described in detail, the scope of the present invention is not limited to this description, and it will be apparent to those skilled in the art that and various modifications and alterations are possible without departing from the technical spirit of the present invention as set forth in the claims.

### Industrial Applicability

The present invention is intended to overcome the limitations of conventional treatment of kidney disease such as acute renal failure, and to develop a more effective therapeutic agent. Currently, studies on identification of kidney stem cells, identification of kidney stem cells for application as therapeutic agents, and the applicability thereof for clinical use are still insufficient, and there is a need for the development of such kidney stem cells. Kidney tissue-derived stem cells or organoids according to the present invention are easy to apply for treatment, have an excellent ability to differentiate into kidney cells, are less likely to form tumors, and have an excellent ability to regenerate damaged tissue when injected directly into lesions, and thus they may be used very effectively for the prevention or treatment of kidney disease.

Sequence Listing Free Test

SEQ ID NO. 1:
















































SEQ ID NO. 2:
MDVLPMCSIF QELQIVHETG YFSALPSLEE YWQQTCLELE
RYLQSEPCYV SASEIKFDSQ
EDLWTKIILA REKKEESELK ISSSPPEDTL ISPSFCYNLE TNSLNSDVSS
ESSDSSEELS
PTAKFTSDPI GEVLVSSGKL SSSVTSTPPS SPELSREPSQ LWGCVPGELP
SPGKVRSGTS
GKPGDKGNGD ASPDGRRRVH RCHFNGCRKV YTKSSHLKAH
QRTHTGEKPY RCSWEGCEWR
FARSDELTRH FRKHTGAKPF KCSHCDRCFS RSDHLALHMK RHL
SEQ ID NO. 3: GGTGAGCCTGGCCTTATGTGAATA
SEQ ID NO. 4: CACCACCATCCTGCACCTCC
SEQ ID NO. 5: TACTCTTTCCTTCAGGCAGTGA
SEQ ID NO. 6: GATCGAGGCAAGTGCATGG
SEQ ID NO. 7: CACCTCCACAAGAATGAAAGCG
SEQ ID NO. 8: CTCCGCCTCGATGTAGTGC
SEQ ID NO. 9: AACCTTGGAGTGAAGGATCGC
SEQ ID NO. 10: GTAGGAGAGCCTATTGGAGATGT
SEQ ID NO. 11: CTCAACATTTCCAATCCGACCC
SEQ ID NO. 12: GGCATCCTTGCTCTTAGTGGG
SEQ ID NO. 13: GAGAGCCAGCCTACCATCC
SEQ ID NO. 14: GGGTCCTCGTGTTTGAAGGAA

SEQ ID NO. 15: AAGTTCGGTTTTCGCTCCAGG

SEQ ID NO. 16: ACACCCCGGAGAGGTTCTG

SEQ ID NO. 17: CTCGGCCATTCGTACATGGAA

SEQ ID NO. 18: GGATACCTCTGCACCGTAGC

SEQ ID NO. 19: GCGTCAGGGAGATGGTAAAG

SEQ ID NO. 20: CATCAGGGAAACAGTTGCAG

SEQ ID NO. 21: CGCTAAGAATCCGCTGGTGAAG

SEQ ID NO. 22: GGATCTTGACGAAGCAGTCGTT

SEQ ID NO. 23: CCTCGGGTCAGTTTGAGCTG

SEQ ID NO. 24: CCTTGAGGCACACTTTGAAGTA

## Claims

1. A pharmaceutical composition for preventing or treating kidney disease containing, as an active ingredient, kidney tissue-derived stem cells expressing Lrigl (leucine-rich repeats and immunoglobulin-like domains 1) protein or a gene encoding the same.

2. The pharmaceutical composition of claim 1, wherein the kidney tissue-derived stem cells further express Klf6 (Krueppel-like factor 6) protein or a gene encoding the same.

3. The pharmaceutical composition of claim 1, wherein the kidney disease is acute kidney injury (AKI) or chronic kidney disease (CKD).

4. A kidney organoid comprising kidney tissue-derived stem cells expressing Lrigl protein or a gene encoding the same.

5. The kidney organoid of claim 4, wherein the kidney tissue-derived stem cells further express Klf6 (Krueppel-like factor 6) protein or a gene encoding the same.

6. A pharmaceutical composition for preventing or treating kidney disease containing the kidney organoid of claim 4 or 5 as an active ingredient.

7. A method for producing kidney organoids comprising steps of:
(a) isolating cells expressing Lrigl (leucine-rich repeats and immunoglobulin-like domains 1) protein or a gene encoding the same from kidney epithelial cells isolated from a subject of interest;
(b) culturing the cells expressing the Lrigl protein or the gene encoding the same; and
(c) forming organoids from the cultured cells in Matrigel.

8. The method of claim 7, wherein the cells isolated in step (a) further express Klf6 (Krueppel-like factor 6) protein or a gene encoding the same.

9. The method of claim 7, wherein step (b) of culturing the cells expressing the gene is performed using a cell culture medium containing fetal bovine serum, a growth factor, and an antibiotic.

10. The method of claim 7, wherein step (c) of forming the organoids is performed using a cell culture medium containing a B27 supplement, a conditioned medium, a growth factor, N-acetylcysteine, and an ALK 5 (TGFβ kinase/activin receptor-like kinase) inhibitor.

11. The method of claim 10, wherein the conditioned medium is at least one selected from the group consisting of Wnt3a conditioned medium, Noggin conditioned medium, and Rspo1 conditioned medium.

12. The method of claim 7, wherein the Matrigel is a growth factor-reduced Matrigel.

13. A composition for detecting kidney tissue-derived stem cells containing an agent for measuring an expression level of Lrigl (leucine-rich repeats and immunoglobulin-like domains 1) protein or a gene encoding the same.

14. The composition of claim 13, further containing an agent for measuring an expression level of Klf6 (Krueppel-like factor 6) protein or a gene encoding the same.

15. The composition of claim 13, wherein the agent for measuring the expression level of the gene is at least one selected from the group consisting of primers, probes, and antisense oligonucleotides, which bind specifically to the gene.

16. The composition of claim 13, wherein the agent for measuring the expression level of the protein is at least one selected from the group consisting of antibodies, oligopeptides, ligands, peptide nucleic acids (PNAs), and aptamers, which bind specifically to the protein.

17. A kit for detecting kidney tissue-derived stem cells comprising the composition of any one of claims 13 to 16.

18. A method for detecting kidney tissue-derived stem cells comprising a step of measuring an expression level of Lrigl (leucine-rich repeats and immunoglobulin-like domains 1) protein or a gene encoding the same from a biological sample isolated from a subject of interest.

19. The method of claim 18, wherein an expression level of Klf6 (Krueppel-like factor 6) protein or a gene encoding the same from the biological sample is further measured in the step of measuring the expression level.

20. The method of claim 18, wherein the expression level of the gene is measured by at least one selected from the group consisting of primers, probes, and antisense oligonucleotides, which bind specifically to the gene.

21. The method of claim 18, wherein the expression level of the protein is measured by at least one selected from the group consisting of antibodies, oligopeptides, ligands, PNAs, and aptamers, which bind specifically to the protein.

22. The method of claim 18, further comprising a step of detecting, as the kidney tissue-derived stem cells, cells in which the expression level of Lrigl (leucine-rich repeats and immunoglobulin-like domains 1) protein or the gene encoding the same is higher than a control group.

23. A method for isolating kidney tissue-derived stem cells comprising a step of isolating cells, which express Lrigl (leucine-rich repeats and immunoglobulin-like domains 1) protein or a gene encoding the same, from a biological sample isolated from a subject of interest.

24. The method of claim 23, wherein the isolated cells further express Klf6 (Krueppel-like factor 6) protein or a gene encoding the same.

25. The method of claim 23, wherein the step of isolating is performed by magnetic activated cell sorting (MACS) or flow cytometry analysis.

26. A method for culturing kidney tissue-derived stem cells comprising steps of:
isolating kidney tissue-derived stem cells expressing Lrigl (leucine-rich repeats and immunoglobulin-like domains 1) protein or a gene encoding the same; and
culturing the isolated kidney tissue-derived stem cells.

27. The method of claim 26, wherein the isolated cells further express Klf6 (Krueppel-like factor 6) protein or a gene encoding the same.

28. A method for producing an animal model for screening a cell therapy product for preventing or treating kidney disease, the method comprising:
inducing kidney injury in an animal in which a gene encoding Lrigl (leucine-rich repeats and immunoglobulin-like domains 1) is conditionally expressed by a CreERT2-LoxP system; and
inducing expression of a gene of interest in the animal by treatment with an estrogen antagonist.

29. The method of claim 28, wherein the step of inducing kidney injury is performed by any one selected from the group consisting of intraperitoneal administration of folic acid, induction of ischemia/reperfusion injury, and induction of unilateral ureteral obstruction.

30. An animal model for screening a cell therapy product for preventing or treating kidney disease, produced according to the method of claim 28.
